(19) Europäisches Patentamt
European Patent Office
Office européen des brevets

(11) Publication number: **0 140 958**
**B1**

# (12) EUROPEAN PATENT SPECIFICATION

(45) Date of publication of patent specification: **20.12.89**

(21) Application number: **84901890.8**

(22) Date of filing: **20.04.84**

(86) International application number:
**PCT/US84/00608**

(87) International publication number:
**WO 84/04246 08.11.84 Gazette 84/26**

(51) Int. Cl.⁴: **A 61 K 31/15**, A 61 K 31/66, A 61 K 31/71, A 61 K 31/16, A 61 K 31/70, A 61 K 31/165

## (54) ONCOLYTIC DRUG COMBINATIONS.

(30) Priority: **21.04.83 US 487368**

(43) Date of publication of application:
**15.05.85 Bulletin 85/20**

(45) Publication of the grant of the patent:
**20.12.89 Bulletin 89/51**

(84) Designated Contracting States:
**AT BE CH DE FR GB LI NL SE**

(56) References cited:

**Elford et al., Cancer Research, vol. 39, pages 844-851, published March 1979**

**Wampler et al., Journal of Medicinal Chemistry, vol. 22, no. 5, pages 589-592, published May 1979**

**Elford et al., Advances in Enzyme Regulation, vol. 19, pages 151-168, published 1981**

**Physicians Desk Reference, 33rd. edition, pages 565, 566, 1108, 1109, published 1979**

(73) Proprietor: **ELFORD, Howard L.**
**3313 Gloucester Road**
**Richmond, VA 23227 (US)**

(73) Proprietor: **WAMPLER, Galen L.**
**6938 Chamberlayne Road**
**Mechanicsville, VA 23111 (US)**

(73) Proprietor: **VAN 'T RIET, Bartholomeus**
**3419 Noble Avenue**
**Richmond, VA 23222 (US)**

(72) Inventor: **ELFORD, Howard L.**
**3313 Gloucester Road**
**Richmond, VA 23227 (US)**
Inventor: **WAMPLER, Galen L.**
**6938 Chamberlayne Road**
**Mechanicsville, VA 23111 (US)**
Inventor: **VAN 'T RIET, Bartholomeus**
**3419 Noble Avenue**
**Richmond, VA 23222 (US)**

(74) Representative: **Werffeli, Heinz R., Dipl.-Ing.**
**Postfach 275 Waldgartenstrasse 12**
**CH-8125 Zürich-Zollikerberg (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 140 958 B1

## Description

The present invention concerns a pharmaceutical product for treating leukemia in mammals.

Doxorubicin (adriamycin) is effective against a broad spectrum of tumors, including acute leukemias and malignant lymphomas. It is also present as one of the ingredients in the multiple drug therapy of non-Hodgkins' lymphomas, carcinomas of the breast and small cell carcinomas and is particularly beneficial in such therapy against a wide variety of sarcomas including osteogenic, Ewing's and soft tissue. It is one of the most active oncolytic agents for treatment of metastatic adrenocarcinomas of the breast, carcinoma of the bladder, bronchogenic carcinoma and neuroblastoma. The activity against solid tumors coupled with good activity against leukemias and lymphomas makes doxorubicin a unique drug. It would probably be the most widely used oncolytic agent today were it not for its toxicity, which toxicity is unrelated to its oncolytic activity. The chief side effect which limits the utility of doxorubicin is serious, irreversible myocardial toxicity with delayed congestive heart failure often unresponsive to any cardiac supportive therapy. The cardiotoxicity is apparently cumulative.

A great deal of research has been devoted to finding ways to decrease the cartiotoxicity of doxorubicin. The chief strategy so far has been to modify the doxorubicin structure chemically. The result of such experimental work, however, has not yielded any compound with improved therapeutic ratio sufficient to replace doxorubicin in the clinic. As a consequence, doxorubicin is chiefly employed as one of several drugs used in a shot gun or multiple drug therapy where it can be administered at drug levels well below the cardiotoxic dose.

The synthesis and structure of a group of di- and trihydroxybenzohydroxamic acids are disclosed in among other publications Elford et al., *Can. Res., 39,* 844 (1979), van't Riet et al., *J. Med. Chem., 22,* 589 (1979) and Elford et al., *Advances in Enzyme Regulation, 19,* 151 (1981). The compounds show excellent activity against L1210 leukemia. The most active compounds are in general those lacking orthohydroxyl and of these 3,4-dihydroxybenzohydroxamic acid appeared to be the most active. The possibility of combining one of these di- or triohydroxybenzohydroxamic acids with another oncolytic agent is not mentioned in the references. In particular, there is nothing in the references that indicates that a ribonucleotide reductase inhibitor would be suitable candidate for combination with doxorubicin to provide an antineoplastic therapeutic combination of enhanced activity and lowered toxicity.

It is an object of this invention to provide such therapeutic combinations enabling the administration of doxorubicin at a reduced dose, thereby lessening the cardiotoxic effects with no loss in oncolytic activity.

In fulfilment of the above and other objects, this invention provides a pharmaceutical product comprising a combination of dioxorubicin or its hydrochloride or a doxorubicin derivative with comparable antitumor effect and toxicity and a hydroxybenzohydroxamic acid of the formula:

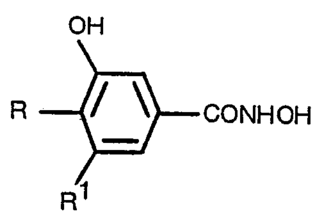

(I)

wherein one of R and $R^1$ is OH and the other is H or OH.

Hydroxybenzohydroxamic acids represented by the above formula include 3,4-dihydroxybenzohydroxamic acid, 3,5-dihydroxybenzohydroxamic acid and 3,4,5-trihydroxybenzohydroxamic acid.

Doxorubicin and a hydroxybenzohydroxamic acid of formula I are administered intravenously to the patient suffering from neoplastic disease. Vials containing a lyophilized mixture of doxorubicin and a hydroxybenzohydroxamic acid are reconstituted with preferably isotonic saline solution USP containing 0.9% sodium chloride. Doxorubicin is usually present as the hydrochloride salt. Sterile water may also be used to reconstitute the lyophilized mixture. The reconstituted solutions are then administered to the patient. The usual dosage level for doxorubicin hydrochloride is 60—75 mg/$m^2$ or 100—130 mg per 75 kg (average weight for humans). These dosage levels are equivalent to dosages of 1.33—1.73 mg/kg of mammalian body weight. The hydroxybenzohydroxamic acid according to formula I above are administered at a rate of 12—26 mg/kg of mammalian body weight or 900—1950 mg per patient, 530—1150 mg/$m^2$.

Ordinarily, doxorubicin hydrochloride is administered at the higher dose levels 60—75 mg/$m^2$ only once every 21 days although an alternate regimen is available in which half the dosage is given on three successive days, followed by a 3 week waiting period. The benzohydroxamic acids of formula I are relatively nontoxic and can be administered daily, if desired. Using the pharmaceutical product according to the application, dose levels of doxorubicin 20—60% of the usual therapeutic dose can be employed (12—15 to 36—45 mg/$m^2$; 20—75 mg per 75 kg person) coupled with 12—26 mg/kg, or 530—1150 mg/$m^2$ of the benzohydroxamic acid of formula I. It is an advantage of the novel combination of oncolytic drugs that, since the doxorubicin hydrochloride can be administered in the combination in far lower doses (as low as

20% of the usual dose) with equal therapeutic effect, it can also be administered more often without any increase in cumulative toxicity. As will be demonstrated below, it is possible in a therapeutic combination to administer as little as .27 mg/kg or 12 mg/m$^2$ up to 1.0 mg/kg of doxorubicin hydrochloride accompanied by 12—26 mg/kg of a hydroxybenzohydroxamic acid of formula I above with comparable therapeutic results.

For intravenous administration a lyophilized vial containing for example, 50 mg of doxorubicin hydrochloride can be reconstituted with 25 ml of isotonic saline and for example from 500—1500 mg of a benzhydroxamic acid dissolved therein. Naturally, if more isotonic saline is required to give a clear solution at the higher levels of benzohydroxamic acid, more saline can be added without affecting the efficacy of the treatment. Alternatively, the doxorubicin hydrochloride and hydroxybenzohydroxamic acid of formula I can be dissolved in water and lyophilized. The vials containing the combined lyophilized solids are then reconstituted with normal saline or sterile water to give the desired solution ready for intravenous injection.

After administering the combination of doxorubicin and a hydroxybenzohydroxamic acid of formula I once every three weeks or at lower doses on successive days, it is usual to follow this combined dosage by administration of the benzohydroxamic acid alone at the same dose level daily or on alternate days for a brief period of time. Because of its lower toxicity, it is possible to administer far larger quantities of the benzohydroxamic acid by itself while doxorubicin hydrochloride is administered far less frequently. The entire regimen can be repeated every three weeks provided toxic levels of doxorubicin are not found.

As evidence of the efficacy of the novel pharmaceutical product the combination of drugs were administered to mice bearing a transplanted tumor, L-1210 leukemia. Synergistic results were obtained by the combined therapy of doxorubicin and hydroxybenzohydroxamic acid, specifically 3,4-dihydroxybenzohydroxamic acid, in this experiment, as set forth below in Tables I and II. In this procedure, L-1210 leukemia was maintained by weekly passage of $10^5$ L-1210 cells intraperitoneally into DBA/2 mice. Diluted ascitic fluid, 0.1 ml. ($10^5$ cells), was administered up to female B6D2F$_1$ mice weighing about 20 g. Drugs were administered intraperitoneally to the animals. Injections containing doxorubicin were given only on day 2 and the 3,4-dihydroxybenzohydroxamic acid was given on days 2, 3 and 4.

In the Tables, column 1 gives the medication, either a control group receiving nothing, doxorubicin hydrochloride by itself, 3,4-hydroxybenzohydroxamic acid by itself or various combinations of the two drugs, column 2 gives the dosage in mg./kg., column 3 the mean life span, column 4 the percent increase in life span over controls and column 5 the percent cures.

TABLE 1

| Medication | Dosage Mg./Kg. | Mean Life Span | Percent Increase In Life Span | Percent Cures* |
|---|---|---|---|---|
| Control | — | 10.9 | — | 0 |
| Doxorubicin hydrochloride | 6 | 14.5 | 33 | 0 |
| | 17 | 28.5 | 161 | 0 |
| | 29 | 27.75 | 155 | 0 |
| 3,4-dihydroxy benzohydroxamic acid | 275 | 12.75 | 17 | 0 |
| | 430 | 13.0 | 19 | 0 |
| | 645 | 11.0 | 1 | 0 |
| doxorubicin hydrochloride plus 3,4-dihydroxybenzohydroxamic acid | 6 + 275 | 28.25 | 159 | 25 |
| | 17 + 275 | 31.25 | 187 | 0 |
| | 29 + 275 | 12.75 | 17 | 0 |
| | 6 + 430 | 28.25 | 159 | 25 |
| | 17 + 430 | 39.0 | 258 | 25 |
| | 29 + 430 | 8.5 | −22 | 0 |
| | 6 + 645 | 31.25 | 187 | 25 |
| | 17 + 645 | 24.0 | 120 | 0 |
| | 29 + 645 | 11.5 | 6 | 0 |

* Survivors calculated at day 60

** Four mice per group

TABLE II

| Medication | Dosage Mg./Kg. | Mean Life Span | Percent Increase In Life Span | Percent Cures* |
|---|---|---|---|---|
| Control** | — | 9.1 | — | 0 |
| Doxorubicin HCl | 6 | 13.3 | 46 | 0 |
| | 17 | 16 | 76 | 0 |
| | 29 | 26.3 | 189 | 33 |
| 3,4-dihydroxybenzohydroxamic acid | 430 | 12 | 32 | 0 |
| | 645 | 13.6 | 49 | 0 |
| Doxorubicin hydrochloride plus 3,4-dihydroxybenzohydroxamic acid | 6 + 430 | 13.7 | 51 | 0 |
| | 17 + 430 | 32.7 | 259 | 67 |
| | 29 + 430 | 33.7 | 270 | 67 |
| | 6 + 645 | 32 | 252 | 67 |
| | 17 + 645 | 27.7 | 204 | 33 |
| | 29 + 645 | 9.7 | 7 | 0 |

* Survivors calculated on day 35

** Three mice per group

As can be seen from the above Tables, 3,4-dihydroxybenzohydroxamic acid at any dose level gives very little increase in life span and in one experiment (Table I — 645 mg./kg.) life span was no better than control. Doxorubicin by itself gave substantial increase in life span with one survivor at the highest dose level tested. However, combinations of lower levels of doxorubicin with 275 or 430 mg./kg. of 3,4-dihydroxybenzohydroxamic acid gave substantial increases in life span plus survivors when compared with the dose level of doxorubicin by itself or of 3,4-dihydroxybenzohydroxamic acid by itself. These increased life spans tended to fall off at the higher dose levels for doxorubicin indicating toxicity. The data clearly indicates that doxorubicin hydrochloride can be administered at dose levels as low as 20% of the usual dose level (6 mg. vs. 29 mg.) with varying amounts of 3,4-dihydroxybenzohydroxamic acids with no loss in efficacy vs. L-1210 leukemia.

Doxorubicin derivatives with comparable antitumor effect and toxicity can be employed in place of doxorubicin in the novel pharmaceutical products and formulations — see chapter by Arcarnone in *Anticancer Agents Based on Natural Products Models* (Ed. Cassady and Douros, Academia Press 1980 — New York) for lists of such derivatives.

## Claims

1. A pharmaceutical product comprising a combination of doxorubicin or its hydrochloride or a doxorubicin derivative with comparable antitumor effect and toxicity and a hydroxybenzohydroxamic acid of the formula

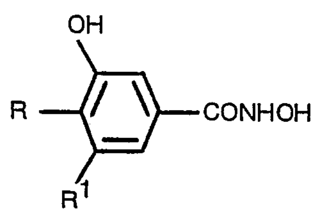

(I)

wherein one of R and $R^1$ is OH and the other is H or OH.

2. A pharmaceutical product of intraveneous injection according to Claim 1 comprising an isotonic saline or sterile water solution containing one part by weight of the doxorubicin hydrochloride and from 5 to 100 parts by weight of the hydroxybenzohydroxamic acid of the formula

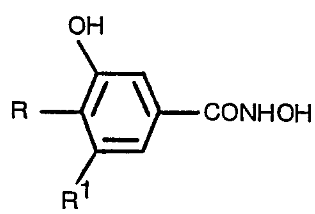

(I)

wherein one of R and $R^1$ is OH and the other is H or OH.

3. A pharmaceutical product according to Claim 1 or 2 in which 3,4-dihydroxybenzohydroxamic acid is present.

4. A pharmaceutical product containing a combination according to any one of the Claims 1 to 3 for treating leukemia in mammals.

5. A pharmaceutical product according to any one of the Claims 1 to 3 as a combined preparation for sequential use in a method for treating leukemia in mammals in which the administration of an effective amount of the combination of doxorubicin and hydroxybenzohydroxamic acid is followed by daily administration of the hydroxybenzohydroxamic acid alone.

**Patentansprüche**

1. Pharmazeutisches Produkt, enthaltend eine Kombination von Doxorubicin oder seiner Hydrochloride oder eines Doxorubicinderivats mit vergleichbarer Antitumorwirkung und Giftigkeit, und einer Hydroxybenzhydroxamsäure der Formel

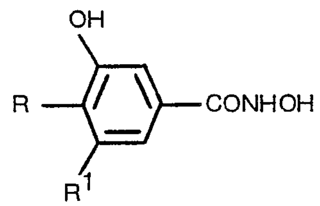

(I)

worin R oder $R^1$ Oh und das andere H oder OH bedeutet.

2. Pharmazeutisches Produkt für intravenöse Injektion nach Anspruch 1, enthaltend eine isotonische Salzlösung, oder sterile Wasserlösung enthaltend einen Gewichtsteil Doxorubicinhydrochlorid und 1 bis 100 Gewichtsteile Hydroxybenzhydroxyamsäure der Formel

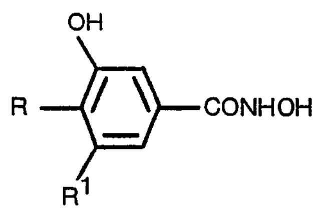

(I)

worin R oder $R^1$ OH und das andere H oder OH bedeutet.

3. Pharmazeutisches Produkt nach Anspruch 1 oder 2, enthaltend 3,4-Dihdroxybenzhydroxamsäure.

4. Pharmazeutisches Produkt enthaltend eine Kombination nach einem der Ansprüche 1 bis 3 zur Behandlung von Leukämie bei Säugetieren.

5. Pharmazeutisches Produkt nach einem der Ansprüche 1 bis 3, als kombiniertes Präparat zur aufeinanderfolgenden Anwendung bei einem Verfarhen zur Behandlung von Leukämie bei Säugetieren, bei welchem die Eingabe einer wirksamen Menge der Kombination von Doxorubicin und Hydroxybenzhydroxansäure gefolgt wird von einer täglichen Dosis der Hydroxybenzhydroxansäure allein.

**Revendications**

1. Un produit pharmaceutique composé de l'association de doxorubicine ou de son hydrochlorydrate ou d'un dérivé de doxorubicine d'activité antitumorale et de toxicité similaire et d'un acide hydroxybenzohydroxamique de formule:

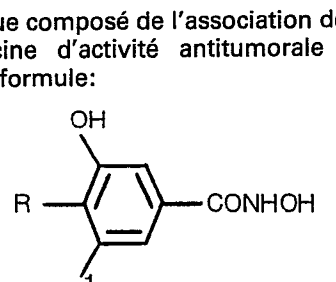

(I)

où l'un des substituants R ou $R_1$ est un groupement —OH et l'autre —H ou —OH.

2. Un produit pharmaceutique selon la revendication 1 injectable par voie intraveineuse constitué d'une solution, dans du soluté salin isotonique ou dans de l'eau stérile, contenant une partie un poids d'hydrochlorydrate de doxorubicine et de 1 à 100 partie en poids d'acide hydroxybenzohydroxamique de formule:

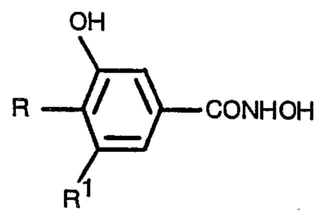

(I)

où l'un des substituants R ou $R_1$ est un groupement — OH et l'autre — H ou — OH.

3. Un produit pharmaceutique selon la revendication 1 ou 2, contenant de l'acide 3,4-dihydroxybenzohydroxamique.

4. Un produit pharmaceutique composé d'une des associations selon l'une des revendications 1 à 3 proposé dans le traitement de leucémie chez les mammiferes.

5. Un produit pharmaceutique selon l'une des revendications 1 à 3 comme association de produit pour utilisation séquentielle dans une méthode de traitement de leucémie chez les mammiféres dans laquelle l'administration d'une dose efficace de l'association de doxorubicine et d'acide hydroxybenzohydroxamique est poursuivie par l'administration journalière d'acide hydroxybenzohydroxamique seul.